# EUROPEAN PATENT APPLICATION

(11) **EP 4 282 401 A1**
(43) Date of publication of application: **29.11.2023**
(21) Application number: 21920748.7
(22) Date of filing: 29.11.2021
(51) Int. Cl.: A61K 9/08, A61K 45/00, A61K 47/32, A61K 47/38, A61K 47/26, A61K 47/10, A61P 27/02, A61P 29/00

(54) **OPHTHALMIC PREPARATION ADMINISTERED BY EYE DROPS AND USED FOR PREVENTING AND TREATING DRY MACULAR DEGENERATION AND RETINAL LIGHT DAMAGE**

(30) Priority: 22.01.2021 CN 202110091088
(71) Applicant: Chengdu Ruimu Biopharmaceuticals Co., Ltd., Chengdu, Sichuan 611135 (CN)
(72) Inventor: DONG, Qing, Chengdu, Sichuan 611135 (CN); XUE, Lubing, Chengdu, Sichuan 611135 (CN)
(74) Representative: Altmann Stößel Dick Patentanwälte PartG mbB
(86) International application number: PCT/CN2021/134150
(87) International publication number: WO 2022/156372

(57) **Abstract**

An ophthalmic preparation used for preventing and treating dry macular degeneration and retinal light damage, which is a preparation composed of an active ingredient for treating an ocular disease and an ophthalmic preparation carrier or adjuvant. The active ingredient for treating the ocular disease is an adenylate-activated protein kinase activator and/or an anti-inflammatory active ingredient. The ophthalmic preparation carrier or adjuvant contains the following components: a surfactant, an ionic macromolecule, and a solvent. The ophthalmic preparation can be administered by eye drops for carrying (wrapping) the adenylate-activated protein kinase activator and/or the anti-inflammatory active ingredient through the anterior eye segment, and delivering same to a posterior eye segment for preventing and treating dry macular degeneration and retinal light damage, and has extremely excellent clinical use value and very positive social significance.

## Description

### Technical field

The present invention belongs to the field of ophthalmic medicaments, and in particular relates to an ophthalmic preparation for preventing and treating dry macular degeneration as well as preventing retinal light damage by eye drop administration.

### Background technology

There are many patients with fundus diseases, and only in China, the number of patients has exceeded tens of millions. With the aging society and the popularity of electronic products, the incidence rate will increase year by year; common fundus diseases include diabetic Macular edema, diabetic retinopathy (DR), age-related macular diseases, retinal vein occlusion (RVO), pathological myopia, geographic atrophy, eye tumors, non-infectious endophthalmitis, etc., which may lead to decreased vision or even blindness, seriously affecting people's quality of life.

Age-related macular disease (AMD) can be divided into dry and wet. Dry AMD is characterized by geographic atrophy, while wet AMD is characterized by choroidal neovascularization (CNV). In the investigation of AMD treatment, Chen et al. had analyzed 68205 cases of diabetes patients for 13 years (Taiwan, 2001-2013), and found that the risk of AMD in patients with type 2 diabetes who took metformin (Metformin, CAS No. 657-24-9) orally was significantly lower than that in patients who did not take metformin; the higher the dose of metformin, the more obvious the reduction of AMD risk (Yu-Yen Chen et al., Association between Metformin and a Lower Risk of Age-Related Macular Degeneration in Patients with Type 2 Diabetes, Journal of Ophthalmology, Volume 2019, Article ID 1649156, 9 pages). Brown et al. had tracked 7788 cases of diabetes patients (Florida, USA) for 7 years and found that oral metformin may have therapeutic effect on the occurrence or development of AMD; it had also been found that compared with the patients who did not take metformin, the high-dose group (>1110 g metformin for 2 years) could reduce the incidence of open-angle glaucoma to the greatest extent (25%) (Emily E. Brown et al., The Common Antidiabetic Drug Metformin Reduces Odds of Developing Age-Related Macular Degeneration, IOVS, April 2019, Vol. 60(5): 1470-77). Doxycycline (CAS No. 564-25-0) has been used as a broad-spectrum antibiotic for many years in clinical practice, usually with an oral dose of 100 mg/day. In recent years, many studies have shown that low-dose doxycycline (20-40 mg/day) has obvious effects on chronic inflammation such as rosacea (R.D. Caprio et al., Anti-Inflammatory Properties of Low and High Doxycycline Doses: An in Vitro Study, Mediators of Inflammation, Vol. 2015, Article ID 329418, 10 pages). A randomized, double-blind, placebo-controlled phase III clinical trial (TOGA; NCT01782989) has shown that doxycycline capsule, with the trade name ORACEA^{®} (40 mg doxycycline), also has a significant effect on the improvement of dry age-related macular lesions with fundus geographic atrophy, once a day for 24 months.

Photochemical damage of retina can cause photoreceptor cell apoptosis and retinal degeneration, which can lead to visual loss for severe cases. Xu et al. have studied whether the injection of metformin has a protective effect on the eyes of animals. Before the light damage experiment, injecting metformin into the vitreous cavity of mice (up to 500 mg/kg/day, for 7 days) can almost completely prevent the light damage, and obviously protect the retinal structure and photoreceptor cells. The results have shown that metformin locally activates AMPK (adenosine mono-phosphate activated protein kinase) in the retina of eyes, which is important for protecting the degeneration of photoreceptors and the retinal pigment epithelium (Xu et al., Stimulation of AMPK prevents degeneration of photoreceptors and the retinal pigment epithelium, PNAS, 2018, 115 (41): 10475-10480).

The above research results have proved the effectiveness of metformin, lipoic acid (CAS No. 62-46-4), resveratrol (CAS No. 501-36-0), doxycycline and other medicaments in the treatment of AMD and the prevention of retinal light damage, but all of them involve oral administration or intravitreal injection and other drug delivery pathways.

There are usually three ways for clinical ophthalmic administration: (1) Conjunctival sac administration (eye drops): medicaments can diffuse and distribute to the iris and Ciliary body after entering the anterior chamber water through the cornea, but the barrier effect of the lens and vitreous membrane makes it difficult for drugs to enter the lens and vitreous body; (2) Eye injection administration: it includes subconjunctival injection, anterior chamber injection, vitreous injection, retroocular injection, and orbital injection. Injecting medication can make the medicament directly reach the treatment site, but injection is traumatic and poses potential risks, such as anterior chamber injection can cause pain, photophobia, tearing, anterior chamber turbidity, bleeding, corneal endothelial cell damage, traumatic cataracts, etc; vitreous injection can cause lens opacity, vitreous organization, retinal/optic nerve damage, etc; (3) Systemic administration includes oral administration and intravenous administration: medicaments in the body generally gather in the liver, kidney or lung, and are blocked by the blood retinal barrier (BRB), so the concentration reaching the eye tissue is lower. Meanwhile, the whole body, especially the main organs, suffer unnecessary side effects. Especially, oral administration of doxycycline may cause systemic side effects, including skin complications (including photosensitivity reactions and skin damage), gastrointestinal side effects (vomiting, diarrhea, dyspepsia), and adverse reactions affecting bone and tooth growth.

At present, in order to allow medicaments to pass through the eye barrier in clinical practice, technical means such as eyeball intravitreal injections (IVI) or implant (ophthalmic insert) are usually used to deliver medicaments to the vitreous body of the patients for treating posterior segment diseases (Pei-Xue Ling, "Ophthalmic Drugs and Preparation Techniques", China Light Industry Press, 2010, P3; Wang et al., Mediators of Inflammation, Vol. 2013, Article ID 780634; Luaces-Rodríguez et al., Pharmaceutics, 2018, 10, 66). However, the operation for intravitreal injection or implant of medicaments is traumatic, that requires specially trained ophthalmologists to perform in sterile environments such as operating rooms; due to the traumatic nature of the operation, complications may occur, such as high intraocular pressure, cataracts, iatrogenic infectious endophthalmitis, vitreous hemorrhage, retinal damage, etc; the requirements of operating conditions and environment are high, and the operation must be carried out in hospitals where the conditions are permitted; the production and usage costs of biopharmaceutical eye injections are high; at the same time, for treatment opportunity, there are also cases of delayed treatment due to medical conditions, resulting in poor flexibility in adjusting dosage regimen (M. HATA et al., RETINA, 37: 1320-1328, 2017).

It has been shown that compared to the methods in the prior art such as intravitreal injection, implantation, oral administration, etc., for the treatment of dry maculopathy and the prevention of retinal light damage, conjunctival sac administration is the most convenient and safe way of eye medication, and the medicaments that are effective for the treatment of AMD and the prevention of retinal light damage, such as metformin, lipoic acid (CAS No. 62-46-4), resveratrol (CAS No. 501-36-0), doxycycline, etc., are developed into special eye drops of low concentration, applied to the eye part, and delivered directly to the fundus, which can avoid trauma to patients, significantly lower the dosage of drugs, and reduce the side effects on human body. Thereby, it is of special significance for the treatment of AMD and the protection of the retina.

However, the cornea has a multi-layer structure, that is roughly divided from the outside to the inside into: an epithelial layer rich in liposomes, a stromal layer rich in water-based components, and an endothelial layer rich in liposomes. After eye drops, the eye drops first contact with the tear layer on the surface of the eye, and then need to cross the epithelial layer, stromal layer, and endothelial layer to reach the posterior segment of the eye. During this process, due to the dilution of tears, the ocular surface barrier of the cornea and conjunctiva, and the barrier of the lens and vitreous body, eye drops often have high concentrations in the tissues of the anterior segment, and it is difficult for the eye drop to enter the posterior segment and achieve effective therapeutic concentrations. Therefore, although the way of conjunctival sac administration is safe, its drug delivery is poor, and thus brings great difficulties in achieving the object of effectively treating fundus diseases.

Totally, the main drug delivery ways in the prior art for the treatment of fundus diseases such as dry maculopathy are difficult to give consideration to both safety and effectiveness. Finding an effective and safe preparation or method for the treatment of fundus diseases is the object that researchers in this field have been striving for.

### Summary of the invention

Compared with intravenous injection and intravitreal injection, the way of eye drop administration has significant advantages in safety and convenience. Inventing ophthalmic preparations that can deliver drugs to the posterior segment of the eye to treat dry maculopathy is a technical problem that needs to be solved urgently in clinical practice, which is of great clinical treatment value and social significance.

The object of the present invention is to provide an ophthalmic preparation for eye drops, which can deliver the active pharmaceutical ingredients for treating an ocular disease to the posterior segment of the eye, to prevent and treat dry maculopathy and retinal light damage.

The present invention provides an ophthalmic preparation for eye drop administration, which is a preparation composed of an active pharmaceutical ingredient for treating an ocular disease as well as an ophthalmic preparation carrier or adjuvant;
the active ingredient for treating the ocular disease is an adenylate-activated protein kinase activator and/or an anti-inflammatory active ingredient;
the ophthalmic preparation carrier or adjuvant contains the following components: a surfactant, an ionic macromolecule, and a solvent.

Further, in the forementioned ophthalmic preparation carrier or adjuvant, the mass ratio of the surfactants to the ionic macromolecule is: (1-100):(0.1-50); the ratio of the surfactant to the solvent is: every 100 mL of the solvent contains 5-3000 mg of the surfactant;
preferably, in the ophthalmic preparation carrier or adjuvant, the mass ratio of the surfactants to the ionic macromolecule is (1-30):(3.5-6); the ratio of the surfactant to the solvent is: every 100 mL of the solvent contains 50-2500 mg of the surfactant;
more further, the surfactant is a non-ionic surfactant;
preferably, the non-ionic surfactant is Spans, Polysorbates, Poloxamer, alkylglucosides, vitamin E polyethylene glycol succinate, sucrose stearates or azone;
more preferably, the non-ionic surfactant is Spans, polysorbates, Poloxamer, alkylglucosides, and sucrose stearates.

Further, the above-mentioned ionic macromolecule is selected from at least one of carboxymethyl cellulose (CMC) and its salts, sodium starch glycolate, hyaluronic acid and its salts, Xanthan gum, alginic acid and its salts, and polyethylene glycol diacetate PEG-(COOH)₂.

Further, in the forementioned ophthalmic preparation carrier or adjuvant, the solvent is a polar solvent, and preferably water.

Further, the ophthalmic preparation carrier or adjuvant also contains the following components: adhesive agents and/or cosolvents.

More further, the adhesive agent is selected from at least one of polyethylene glycol, carbomer, Poloxamer, povidone, hydroxypropyl cellulose, methyl cellulose, hydroxyethyl cellulose, polyvinyl alcohol, Xanthan gum, polyoxyethylene fatty alcohols, hyaluronic acid and its salts or hydroxypropyl methyl cellulose (HPMC); the cosolvent is propylene glycol, glycerol, liquid polyethylene glycol or castor oil; the mass ratio of the adhesive agent to the surfactant is 1 :(0.1-100), and the mass ratio of the cosolvent to the surfactant is (1-10): 1;
more preferably, the mass ratio of the adhesive agent to the surfactant is 1:(0.1-6.25), and the mass ratio of the cosolvent to the surfactant is (4.2-10): 1;

Further, the mass ratio of the surfactant to the active pharmaceutical ingredient for treating the ocular disease is (1-30):(1-2).

Further, the adenylate-activated protein kinase activator is at least one of lipoic acid, stereoisomers of lipoic acid, lipoate, stereoisomer salts of lipoic acid, metformin, metformin salts, resveratrol, resveratrol salts; the anti-inflammatory medicament is at least one of doxycycline, doxycycline salts, tetracycline and tetracycline salts.

More further, the metformin salt is metformin hydrochloride, and the doxycycline salt is doxycycline hydrochloride, and the tetracycline salt is tetracycline hydrochloride.

Further, the forementioned ophthalmic preparation carrier or adjuvent contains nanobodies, which are formed by self-assembly of the components of the ophthalmic preparation carrier or adjuvent; the nanobodies are assembled with active pharmaceutical ingredients for treating the ocular disease.

More further, the above-mentioned nanobody is spherical, with a particle size of 1-100 nm; preferably, the particle size of the nanobody is 5-30 nm.

More further, the above-mentioned preparation also contains nanospheres, which are spherical in shape and have a particle size of 10-2000 nm; the nanospheres are formed by self-assembly of nanobodies; preferably, the particle size of the nanospheres is 100-2000 nm.

The present invention also provides a method for preparing the preparation mentioned above, which comprises the following steps:
(1) The surfactant and/or the adhesive agent is added to the solvent to prepare a solution;
(2) The active pharmaceutical ingredient for treating the ocular disease and/or the cosolvent is dispersed in the solution obtained in step (1), to which is then added the ionic macromolecular or its solution, and the resultant solution is dispersed and mixed to obtain the initial suspension;
(3) The initial suspension obtained in step (2) is stirred and dispersed or homogenized to obtain the preparation;
preferably, in step (2), the active pharmaceutical ingredient is dissolved in an organic solvent, and then dispersed in the solution obtained in step (1).

Further, the dispersion described in step (2) is selected from at least one of mechanical stirring dispersion, magnetic stirring dispersion, vortex shaking dispersion, shear dispersion, homogeneous dispersion, grinding dispersion, and ultrasonic dispersion.

The present invention also provides the use of the preparation mentioned above in the preparation of medicaments for preventing and treating fundus diseases.

Further, the medicament for preventing and treating fundus diseases is a medicament for preventing and treating dry macular degeneration;
or, the medicament for preventing and treating the damage of light to eye photoreceptor cells or retina.

The present invention also provides a method for preventing and treating fundus diseases, i.e. the preparation mentioned above is administrated to the patients or individuals at risk at an effective therapeutic amount.

Further, the forementioned fundus diseases are dry macular degeneration or the damage of light to eye photoreceptor cells or retina.

More further, the pathway used is eye drop administration.

The prior art has proved that metformin hydrochloride has a protective effect on retinal structure and photoreceptor cells of eyes, but it needs to be administered by injection, and it is difficult to for the medicament to achieve an effective therapeutic dose in the posterior segment of the eye by eye drops. The ophthalmic preparation for eye drop administration prepared in the present invention has stable properties and is easy to store. The experiment has confirmed that the preparation can effectively deliver the active pharmaceutical ingredients (e.g. metformin) for treating an ocular disease to the posterior segment of the eye, and reach an effective (expected) concentration in the fundus. Therefore, the treatment and prevention of fundus diseases such as dry macular degeneration as well as the damage of light to eye photoreceptor cells or retina, can be achieved by eye drops. The present invention overcomes the problems of current intravitreal injection, intravitreal injection implant, oral administration and systemic injection, solve the serious complications problems such as intraocular hemorrhage and pain, greatly reduce the pain of patients with fundus diseases, increase medical compliance, improve the life quality of patients and their families, or avoid systemic toxic and side effects caused by systemic medication.

The present invention can avoid complications caused by local injection or implant of the eye.

The preparation developed in the present invention has a small dosage as well as minimal toxic and side effects, and can not only be used as a therapeutic medicament, but also as a control and prevention for ophthalmic diseases.

The preparation of the present invention can meet the needs of long-term administration in clinical practice.

In the eye drop medication system of the present invention, the active pharmaceutical ingredient can be a small molecule drug that has been used in clinical practice and has a clear mechanism of action. The quality is controllable, the product is easy to use, the patient has good compliance, and the doctor can flexibly adjust the medication scheme according to the patient's conditions.

The nanobody used in the present invention refers to a nanoscale spherical aggregate formed by self-assembly of the components of the ophthalmic preparation carrier or adjuvent in a solvent.

The nanosphere used in the present invention refers to spherical self-assembled structures formed by the self-assembly of nanobodies in a solvent.

The solvent used in the present invention refers to a liquid that can dissolve the components of an ophthalmic preparation carrier or adjuvant.

The surfactant used in the present invention refers to a substance that can significantly reduce the surface tension of a liquid; the non-ionic surfactant used in the present invention refers to a surfactant that does not dissociate in water.

The ionic macromolecule used in the present invention refers to a macromolecule with cations or anions.

The "active pharmaceutical ingredient for treating an ocular disease" used in the present invention refers to an active pharmaceutical ingredient (API) that can be used for treating eye diseases, and has already been used as an ophthalmic medicament, as well as the mechanism and target of action indicate that this API can treat eye diseases, but has not been used as an ophthalmic medicament yet.

The eye drop administration of the present invention refers to an administration pathway of dropping the drug solution into the eye, which belongs to the corneal route of administration.

Obviously, based on the above content of the present invention, according to the common technical knowledge and the conventional means in the field, without department from the above basic technical spirits, other various modifications, alternations, or changes can further be made.

With reference to the following specific examples of the embodiments, the above content of the present invention is further illustrated. But it should not be construed that the scope of the above subject matter of the present invention is limited to the following examples. The techniques realized based on the above content of the present invention are all within the scope of the present invention.

### Description of Figures

Figure 1. The transmission electron microscopy image of the sample prepared in Example 2 (with a scale of 500 nm).
Figure 2. The transmission electron microscopy image of the sample prepared in Example 9 after staining (with a scale of 100 nm).
Figure 3. HPLC of Example 2.
Figure 4. HPLC of Example 5.
Figure 5. HPLC of Example 14.
Figure 6. HE staining results of eyeball tissue slices of rats in the control group, the model group of dry macular degeneration, and the treatment group treated with the preparation of the present invention.

### Examples

The reagents or instruments used in the present invention can be obtained from market, and if specific conditions are not specified, they shall be used according to conventional conditions or manufacturer's recommended conditions.

Some instruments and equipment are as follows:
ES225SM-DR(E) electronic analytical balance, Precisa (Switzerland);
DF-101S Heat-collection type thermostatic magnetic agitator, Gongyi Yingyu Rivet Factory (Henan, China);
WH-2 Mini Vortex meter, Shanghai Huxi Analysis Instrument Factory (Shanghai, China);
Disperse machine: T25 easy clean digital, IKA (Germany);
KQ-500 type ultrasonic cleaning machine, Kunshan Ultrasonic Instruments Co., Ltd (Kunshan, China)
JP-010T type ultrasonic cleaning machine, Skymen Cleaning Equipment Shenzhen Co., Ltd.;
AH-NANO Plus High Pressure Homogenizer, AntosNanoTechnology (Suzhou) Co., Ltd. (China);
PM-DK2 Planetary Ball Mill, Droide Instrument and Equipment (Shanghai) Co., Ltd. (Shanghai, China);
Mettler Toledo FE20 pH meter, Mettler-Toledo (Switzerland);
NS-90 Nanoparticle Size Analyzer, Zhuhai Omec Instrument Co., Ltd (Zhuhai, China);
Agilent 1100 HPLC, Agilent Technologies Inc. (USA);
API4000 Triple Quadrupole Mass Spectrometer (Applied Biosystems, USA);
STY-1A Osmotic Pressure Measuring Instrument, Tiandatianfa Technology Corp., Ltd (Tianjin, China).

The method for detecting the properties of the preparation according to the present invention was as follows:

### Method for measuring particle size

1 mL of sample prepared in the Example or Comparative Example was transferred to the sample cell. The detection temperature was set to 40 °C, and then the sample cell was put into the NS-90 Nanoparticle Size Analyzer to start the detection. Each sample was tested three times, and the particle size (in terms of light intensity distribution and % percentage) and polydispersity index (PdI) were represented with the average of three test results.

### Method for measuring osmotic pressure:

The osmotic pressure molar concentration of the solution was measured by measuring its freezing-point depression. Procedures: the probe of STY-1A osmotic pressure measuring instrument was cleaned. Three portions of 100 µL distilled water were respectively added to three sample tubes, and after preheating the instrument, the sample tube containing 100 µL of distilled water was screwed onto the instrument probe, followed by selecting "clean 3 times" and clicking "clean", that was repeated three times. Measuring: After filling in the sample information in the instrument information table, click "testing"; a pipette was used to transfer 100 µL of sample into the sample tube, which was gently screwed onto the instrument, followed by clicking "start" for testing. The test was repeated three times, and the average of the three test results was taken as the result.

### Method for measuring pH value:

The FE20 pH meter was calibrated using pH buffer solutions (pH 4.00, 6.86, and 9.18, respectively). The electrodes were rinsed with pure water, excess water was removed with fiber free paper, and then the electrodes were immersed in the liquid sample to be tested, followed by pressing the reading key to start the measuring. After the reading stabilized, the data obtained was the pH value of the sample.

If the pH value of the test solution was <5 or >9, the solution needed to be adjusted to pH 6-8 with acid or alkali. The commonly used pH regulators were NaOH and HCl, phosphoric acid and phosphate (e.g. sodium dihydrogen phosphate, disodium hydrogen phosphate), citric acid and citrate (e.g. sodium citrate), boric acid and borax; if the osmotic pressure of the obtained solution was detected not to reach an isotonic pressure, an appropriate amount of sodium chloride was added to make the solution reach or close to the isotonic.

### Method for verifying the result of delivering medicaments to the posterior segment of the eye:

Experimental instrument and equipment: High performance liquid chromatography, model: LC-20AD (Shimadzu, Japan); mass spectrometer: model: API4000 triple quadrupole mass spectrometer (Applied Biosystems, USA); chromatographic column: Fortis Pace C18 5 µM, 2.1 × 30 mm (Fortis, UK).

Healthy adult Sprague Dawley (SD) rats were divided into a test group and a control group, with 6 eyes in each group. The test group received eye drops of the ophthalmic preparation prepared in the Example of the present invention, while the control group received the suspension of medicament (2 mg) in 5 mL of physiological saline (vortex shaking prior to use), at a dosage of 20 µL/eye. 0.5 h or 1 h after administration, the animals were euthanized, and the vitreous bodies were quickly collected. The vitreous samples were homogenized and stored at -80 °C. To 10 µL of vitreous homogenate, was added 90 µL of 95% ethanol, and then the solution was mixed under ultrasonic for 2 min, followed by vortex mixing for 1 min, to obtain the vitreous homogenate; to 50 µL of the homogenate, was added 175 µL of methanol, and then the resultant solution was vortex mixed for 3 min, and centrifuged at 4 °C and 12000 rpm for 10 min. The supernatant was filtered using a 0.45 µm needle filter. The filtrate was subjected to LC/MS/MS (Positive ion mode, MRM SCAN) analysis.

### Example 1. Preparing the ophthalmic preparation according to the present invention

Preparation method: according to Table 1, 60 g of CMC-Na (sodium carboxymethyl cellulose, ionic macromolecule) was weighed and added to a glass triangular flask containing 10 mL of pure water, and then stirred for 2 h with a magnetic stirrer to obtain solution 1; 0.3 g of polysorbate 80 (surfactant) and 0.12 g of HPC (low-substituted hydroxypropyl cellulose, adhensive agent) were respectively weighed and added into a glass triangular flask containing 20 mL of pure water, and then stirred with a magnetic stirrer and heated in an water bath at 40 °C for about 1.5 h, to obtain solution 2; 15 mg of metformin hydrochloride was added into solution 2, and then the resultant solution was further heated and stirred for 30 min, which was then added to solution 1, followed by stirring for 30 min, to obtain the mixed solution; the mixed solution was dispersed with a disperser for 5 min at a speed of 9500 rpm, the disperser was turned off, and the solution was rested until the foam disappeared, followed by filtering under reduced pressure with a Büchner funnel, to obtain the dispersed solution; the dispersed solution was transferred to a high-pressure homogenizer, and homogenized at the temperature of 15 ± 5 °C under the pressure of about 400 Bar for 3 min, then homogenized under the increased pressure of >800 Bar for 25 min, followed by under the decreased pressure of 300 Bar for 2 min. The solution was discharged to obtain a colorless and clear solution, which was further filtered under reduced pressure, so as to remove bacteria and mechanical impurities, and obtain a colorless and clear solution after removal of impurities.

Method for adjusting pH value: 0.1 N NaOH was added to adjust pH value to be 6.5.

HPLC detection: Column: ZORBAX Eclipse Plus C18, 4.6 × 100 mm, 3.5 µm; mobile phase A: 40 mM ammonium acetate (pH 5.0), B: methanol; gradient elution, 0-2': 100% A, 20-22': 60% A-40% B, Temp.: 35°C, detection wavelength: 233 nm, flow rate: 0.8 ml/min; injection volume: 10 µL; detection result: 97.4%. Particle size: 11.8 nm (71.6%); PdI: 0.519; the appearance and the content were not obviously changed after storing at room temperature in dark for 1 month.

### Example 2. Preparing the ophthalmic preparation according to the present invention

The preparation method was almost identical to that in Example 1, and the raw materials and their amounts were shown in Table 1. After removal of impurities, a colorless and clear solution was obtained.

Adjusting pH value and osmotic pressure: 1N sodium citrate solution was added to adjust pH to be 6.5; sodium chloride was added to adjust osmotic pressure to be 297 mOsmol/kg.

HPLC detection: column: ZORBAX 300SB-CN, 2.1 × 150 mm, 5 µm; mobile phase: 40 mM KH₂PO₄ (pH 4.5):methanol (75:25) isocratic elution, Temp.: 35 °C, detection wavelength: 233 nm, flow rate: 0.8 ml/min; detection result: 99.1%.

Particle size 21.6 nm (94.4%); PdI: 0.206; no obvious change in the appearance and the content was observed after storing at room temperature in dark for 1 month.

1 h after eye drops, the API concentration in the vitreum of rats was 39.8±16.6 ng/g.

### Example 3. Preparing the ophthalmic preparation according to the present invention

The preparation method was almost identical to that in Example 1, and the raw materials and their amounts were shown in Table 1. After removal of impurities, a colorless and clear solution was obtained. pH test result: 6.9, close to the isotonic, no adjustment required.

The HPLC detection method was the same as that in Example 1, with a detection result of 98.6%.

Particle size: 16.6 nm (98.6%); PdI: 0.227; no obvious change in the appearance and the content was observed after storing at room temperature in dark for 1 month.

### Example 4. Preparing the ophthalmic preparation according to the present invention

The preparation method was almost identical to that in Example 1, and the raw materials and their amounts were shown in Table 1. After removal of impurities, a colorless and clear solution was obtained. pH test result: 6.5, close to the isotonic, no adjustment required.

The HPLC detection method was the same as that in Example 1, with a detection result of 97.8%.

Particle size: 17.1 nm (55.5%), 513 (36.3%); PdI: 0.795; no obvious change in the appearance and the content was observed after storing at room temperature in dark for 1 month.

### Example 5. Preparing the ophthalmic preparation according to the present invention

Preparation method: 60 mg of CMC-Na was weighed and then transferred to a glass triangular flask containing 15 mL of pure water, and then stirred for 2 h with a magnetic stirrer to obtain solution 1; 0.24 g of polysorbate 80 and 0.12 g of HPMC (adhensive agent) were respectively weighed and then added into a glass triangular flask containing 15 mL of pure water, and then stirred with a magnetic stirrer and heated in an water bath at 40 °C for about 3 h, to obtain solution 2; 15 mg of lipoic acid and 1 mL of glycerol (equivalent to 5.25 times the amount of surfactant (w/w)) were added into solution 2, and then the resultant solution was further heated and stirred for 30 min. Subsequently, the solution was added to solution 1, and then stirred for 30 min, to obtain the mixed solution; the mixed solution was dispersed with a disperser for 3 min at a speed of 11,000 rpm, the disperser was turned off, and the solution was rested until the foam disappeared, followed by transferring to a high-pressure homogenizer for homogenization treatment (for conditions, referring to Example 1), to obtain a colorless clear solution. Then, the solution was filtered under reduced pressure to remove bacteria and mechanical impurities, and thus a colorless and clear solution was obtained after removal of impurities;

Method for adjusting pH value and osmotic pressure: 1N sodium citrate solution was added to adjust pH to be 6.3; sodium chloride was added to adjust osmotic pressure to be 294 mOsmol/kg.

HPLC detection: column: ZORBAX Eclipse Plus C18, 4.6 × 100 mm, 3.5 µm; mobile phase A: 0.1% phosphoric acid (pH 3.0), B: methanol-acetonitrile (1:1). Temp.: 35°C, detection wavelength: 215 nm, flow rate: 0.8 ml/min; gradient elution program: 0-5': 60%A-40%B, 28-30': 40%A-60%B; detection result: 97.4%. Particle size: 17.8 nm (98.6%); PdI: 0.222; no obvious change in the appearance and the content was observed after storing at 3-8 °C in dark for 1 month.

0.5 h after eye drops, the API concentration in the vitreum of rats was 52.6±17.9 ng/g.

In the control group, 2 mg of lipoic acid/5mL of normal saline suspension (vortex shaking prior to use) was added dropwise, 20 µL for each eye. In the control group, lipoic acid was not detected in the vitreum of animals 0.5 h after eye drops (below the detection limit, <1 ng/g).

### Example 6. Preparing the ophthalmic preparation according to the present invention

The preparation method and the method for adjusting pH value and osmotic pressure were almost identical to that in Example 1, and the raw materials and their amounts were shown in Table 1. After removal of impurities, a colorless and clear solution was obtained.

The HPLC detection method was the same as that in Example 1, with a detection result of 98.4%.

Detection results: particle size 348 nm (85%), PdI: 0.422.

No obvious change in the appearance and the content was observed after storing at room temperature in dark for 1 month; there was no significant change after 1 month, but a small amount of precipitation appeared after 2 months.

### Example 7. Preparing the ophthalmic preparation according to the present invention

The preparation method and the methods for adjusting pH value and osmotic pressure were almost identical to that in Example 5, and the raw materials and their amounts were shown in Table 1, wherein the dosage of the cosolvent propylene glycol was 6.2 times that of the surfactant (w/w).

The HPLC detection method was the same as that in Example 5, with a detection result of 98.1%; particle size 25.8 nm (87.4%), PdI: 0.317; no obvious change in the appearance and the content was observed after storing at 3-8 °C in dark for 1 month.

### Example 8. Preparing the ophthalmic preparation according to the present invention

The preparation method and the methods for adjusting pH value and osmotic pressure were almost identical to that in Example 5, and the raw materials and their amounts were shown in Table 1, wherein the dosage of the cosolvent propylene glycol was 8.9 times that of the surfactant (w/w).

The HPLC detection method was the same as that in Example 5, with a detection result of 95.2%; particle size 31.5 nm (82.9%), PdI: 0.347; no obvious change in the appearance and the content was observed after storing at 3-8 °C in dark for 1 month.

### Example 9. Preparing the ophthalmic preparation according to the present invention

The preparation method was almost identical to that in Example 1, and the raw materials and their amounts were shown in Table 1. After removal of impurities, a yellowish and clear solution was obtained.

Adjusting pH value and osmotic pressure: 0.1 N NaOH was added to adjust pH to be 6.3; sodium chloride was added to adjust osmotic pressure to be 297 mOsmol/kg;

The detection wavelength used in HPLC was 280 nm, and the remained were the same as that in Example 1, with a detection result of 97.3%; particle size 16.7 nm (98.1%), PdI: 0.225; no change in the appearance and the content was observed after storing at room temperature in dark for 1 month.

0.5 h after eye drops, the API concentration in the vitreum of rats was 66.5 ± 18.1 ng/g.

In the control group, 2 mg of doxycycline/5mL of normal saline suspension (vortex shaking prior to use) was administrated by eye drops, 20 µL for each eye. In the control group, doxycycline was not detected in the vitreum of animals 0.5 h after eye drops (below the detection limit, <1 ng/g).

### Example 10. Preparing the ophthalmic preparation according to the present invention

The preparation method and the methods for adjusting pH value and osmotic pressure were almost identical to that in Example 9, and the raw materials and their amounts were shown in Table 1. After removal of impurities, a yellowish and clear solution was obtained.

The detection method by HPLC was the same as that in Example 9, with a HPLC detection result of 98.2%; particle size 17.2 nm (97.9%), PdI: 0.208; no change in the appearance and the content was observed after storing at room temperature in dark for 1 month.

### Example 11. Preparing the ophthalmic preparation according to the present invention

The preparation method and the methods for adjusting pH value and osmotic pressure were almost identical to that in Example 9. The addition of cosolvent was as follows: 1.5 mL of propylene glycol (equivalent to 10 times the amount of surfactant (w/w)) was weighed and added, together with the surfactant, to the medium water; the resultant solution was dissolved by magnetic stir and heating in a water bath to obtain solution 2; after removal of impurities, a yellowish and clear solution was obtained. The raw materials and their amounts were shown in Table 1.

The HPLC detection method was the same as that in Example 9, with a detection result of 95.2%; particle size 29.7 nm (89.3%), PdI: 0.382; the cottony substance appeared after storing at 3-8 °C in dark for 1 month.

### Example 12. Preparing the ophthalmic preparation according to the present invention

The preparation method and the methods for adjusting pH value and osmotic pressure were almost identical to that in Example 5, and the raw materials and their amounts were shown in Table 1.

The HPLC detection method was the same as that in Example 5, with a detection result of 0.486 mg/mL (metformin) and 0.481 mg/mL (lipoic acid); particle size 18.9 nm + 302.1 nm, PdI: 0.529; there was no change in the appearance and the content after storing at 3-8 °C in dark for 1 month.

0.5 h after eye drops, the API concentration in the vitreum of animals was 86.5 ng/g (lipoic acid) and 69.5 ng/g (metformin).

### Example 13. Preparing the ophthalmic preparation according to the present invention

The preparation method and the methods for adjusting pH value and osmotic pressure were almost identical to that in Example 5, and the raw materials and their amounts were shown in Table 1.

The HPLC detection method was the same as that in Example 5, with a detection result of 0.487 mg/mL (doxycycline) and 0.478 mg/mL (lipoic acid); particle size 20.2 nm + 251.6 nm, PdI: 0.701; no change in the appearance and the content was observed after storing at 3-8 °C in dark for 1 month.

0.5 h after eye drops, the API concentration in the vitreum of animals was 57.3 ng/g (lipoic acid) and 68.4 ng/g (doxycycline).

### Example 14. Preparing the ophthalmic preparation according to the present invention

The preparation method was almost identical to that in Example 5, and the raw materials and their amounts were shown in Table 1, wherein the amount of the cosolvent propylene glycol was 4.5 times that of the surfactant (w/w). pH value of the solution was 6.5, close to the isotonic, and thus there was no adjustment required.

HPLC detection method: column: ZORBAX Eclipse Plus C18, 4.6 × 100 mm, 3.5 µm; mobile phase A: 0.1% phosphoric acid, B: acetonitrile (80:20). Temp.: 35°C, detection wavelength: 306 nm, flow rate: 0.8 ml/min; detection result: 95.7%;

Particle size: 27.5 nm (77.9%); PdI: 0.328; no obvious change in the appearance and the content was observed after storing at 3-8 °C in dark for 1 month.

0.5 h after eye drops, the API concentration in the vitreum of rats was 20.3±9.3 ng/g.

### Example 15. Preparing the ophthalmic preparation according to the present invention

The preparation method was almost identical to that in Example 14, and the raw materials and their amounts were shown in Table 1, wherein the amount of the cosolvent propylene glycol was 4.5 times that of the surfactant (w/w). After removal of impurities, a colorless and clear solution was obtained.
pH value was 6.6, no adjustment required; adjusting the osmotic pressure: sodium chloride was added to adjust osmotic pressure to 293 mOsmol/kg;
The HPLC detection method referred to that in Example 14, with a detection result of 96.1%;
Particle size: 24.5 nm (77.9%); PdI: 0.253; no obvious change in the appearance and the content was observed after storing at 3-8 °C in dark for 1 month.

### Example 16. Preparing the ophthalmic preparation according to the present invention

The preparation method was almost identical to that in Example 14, and the raw materials and their amounts were shown in Table 1, wherein the amount of the cosolvent glycerol was 4.5 times that of the surfactant (w/w). After removal of impurities, a colorless and clear solution was obtained.
pH value was 6.4, and thus there is no need to adjust the pH; adjusting the osmotic pressure: sodium chloride was added to adjust osmotic pressure to 305 mOsmol/kg;
The HPLC detection method referred to that in Example 14, with a detection result of 94.7%;
Particle size: 26.2 nm (75.2%); PdI: 0.325; no obvious change in the appearance and the content was observed after storing at 3-8 °C in dark for 1 month.

### Example 17. Preparing the ophthalmic preparation according to the present invention

The preparation method was almost identical to that in Example 14, and the raw materials and their amounts were shown in Table 1, wherein the amount of the cosolvent propylene glycol was 4.5 times that of the surfactant (w/w). After removal of impurities, a colorless and clear solution was obtained.
Adjusting pH value and osmotic pressure: 0.2 N NaOH was added to adjust pH to 6.2; sodium chloride was added to adjust osmotic pressure to be 305 mOsmol/kg;
The HPLC detection method referred to that in Example 14, with a detection result of 95.3%;
Particle size: 22.7 nm (83.4%); PdI: 0.372; no obvious change in the appearance and the content was observed after storing at 3-8 °C in dark for 1 month.

### Example 18. Preparing the ophthalmic preparation according to the present invention

The preparation method was almost identical to that in Example 9, and the raw materials and their amounts were shown in Table 1, wherein the amount of the cosolvent propylene glycol was 5 times that of the surfactant (w/w). After removal of impurities, a colorless and clear solution was obtained.
Adjusting pH value and osmotic pressure: 0.1 N NaOH was added to adjust pH to be 6.3; sodium chloride was added to adjust osmotic pressure to be 290 mOsmol/kg;
The HPLC detection method referred to that in Example 15, with a detection result of 96.1%;
Particle size: 23.7 nm (84.2%); PdI: 0.323; no obvious change in the appearance and the content was observed after storing at room temperature in dark for 1 month.

0.5 h after eye drops, the API concentration in the vitreum of rats was 62.5 ng/g.

### Example 19. Preparing the ophthalmic preparation according to the present invention

The preparation was carried out with reference to the method in Example 9, and the raw materials and their amounts were shown in Table 1, wherein the amount of the cosolvent propylene glycol was 4.2 times that of the surfactant (w/w). After removal of impurities, a colorless and clear solution was obtained.
Adjusting pH value and osmotic pressure: 0.1 N NaOH was added to adjust pH to be 6.3; sodium chloride was added to adjust osmotic pressure to be 288 mOsmol/kg;
the HPLC detection method referred to that in Example 15, with a detection result of 95.6%; particle size: 24.1 nm (81.5%); PdI: 0.357; no obvious change in the appearance and the content was observed after storing at room temperature in dark for 1 month.

### Example 20. Preparing the ophthalmic preparation according to the present invention

The preparation was carried out with reference to the method in Example 5, and the raw materials and their amounts were shown in Table 1, wherein the amount of the cosolvent propylene glycol was 5.2 times that of the surfactant (w/w). After removal of impurities, a colorless and clear solution was obtained.
Adjusting pH value and osmotic pressure: 0.2 N NaOH was added to adjust pH to be 6.3; sodium chloride was added to adjust osmotic pressure to be 310 mOsmol/kg;
the HPLC detection method referred to that in Example 5, with a detection result of 97.2%; particle size: 27.5 nm (79.6%); PdI: 0.364; particles could be found after storing at room temperature in dark for 1 month.

### Example 21. Preparing the ophthalmic preparation according to the present invention

The preparation was carried out with reference to the method in Example 9, and the raw materials and their amounts were shown in Table 1. After removal of impurities, a yellowish and clear solution was obtained.
Adjusting pH value and osmotic pressure: 0.1 N sodium citrate was added to adjust pH to be 7.0; sodium chloride was added to adjust osmotic pressure to be 302 mOsmol/kg.
HPLC detection: Column: ZORBAX 300SB-CN, 2.1 × 150 mm, 5 µM; mobile phase: 0.1 M ammonium acetate aqueous solution-10 mM triethylamine (pH 8.5):acetonitrile (84:16), isocratic elution; Temp.: 30 °C; detection wavelength: 350 nm; flow rate: 0.8 ml/min; detection result: 98.5%; particle size 13.4 nm (89.5%), PdI: 0.201; after storing at room temperature in dark for 1 month, there was no obvious changes in the appearance and the content.

0.5 h after eye drops, the API concentration in the vitreum of rats was 54.8±19.2 ng/g.

After eye drop administration, the test results of API concentration in the vitreous body of rats indicated that the ophthalmic preparation of the present invention could carry the active pharmaceutical ingredients for treating an ocular disease and cross the barrier of the eyeball structure, and thereby deliver the effective dose of medicaments to the vitreous body by means of conjunctival sac administration (eye drop administration), which could avoid invasive administration routes such as intravitreal injection, and also significantly reduce the total drug amount and the absorption of drugs in the whole body, so as to prevent toxic and side effects.

### Comparative example 1. Preparing the preparation using povidone as excipients

With reference to the preparation method in Example 5, 15 mg of lipoic acid was used as the active pharmaceutical ingredient, while the ionic macromolecule was substituted with povidone K12 (PVP K12), and the adhesive agent with povidone K30 (PVP K30); PEG300 was used as a cosolvent, and the mass of PEG300 was 22 times that of povidone K12; the preparation was obtained by using the amounts shown in Table 1.

### Comparative example 2. The preparation prepared by substituting the ionic macromolecule with povidone

With reference to the preparation method in Example 2, 15 mg of metformin hydrochloride was used as the active pharmaceutical ingredient, while the ionic macromolecule was substituted with povidone K12 (PVP K12); PEG300 was used as the cosolvent, and the mass of PEG300 was 4 times that of the surfactant; the preparation was obtained by using the amounts shown in Table 1.

### Comparative example 3. The preparation prepared by substituting the ionic macromolecule with polyethylene glycol

With reference to the preparation method in Example 15, the ionic macromolecule was substituted with PEG4000; PEG300 was used as the cosolvent, and the mass of PEG300 was 3 times that of the surfactant; the preparation was obtained by using the amounts shown in Table 1.

**Table 1.**

| Exa mple | Surfactant (A) | Ionic macromole cule (B) | Adhesive agent (C) | Amount of API (D) (mg) | Mass ratio of A:B:C: D | Cosol vent | Solve nt |
|---|---|---|---|---|---|---|---|
| 1 | Polysorbate 80 | CMC-Na | hydroxypro pyl cellulose (HPC) | Metformin hydrochlorid e | 20:4:8:1 | | Water 30 mL |
| | | | | 15 mg | | | |
| 2 | Polysorbate 80 | CMC-Na | HPMC | Metformin hydrochlorid e | 20:4:7:1 | | Water 30 mL |
| | | | | 15 mg | | | |
| 3 | Polysorbate 80 | CMC-Na | Poloxamer P-188 | Metformin hydrochlorid e | 25:4:4:1 | | Water 30 mL |
| | | | | 15 mg | | | |
| 4 | Polysorbate 80 | CMC-Na | HPC | Metformin hydrochlorid e | 16:4:8:1 | | Water 30 mL |
| | | | | 15 mg | | | |
| 5 | Polysorbate 80 | CMC-Na | HPMC | Lipoic acid | 16:4:8:1 | Glyce rol | Water 30 mL |
| | | | | 15 mg | | | |
| 6 | Poloxamer P188 | Sodium alginate | PVP K12 | Metformin hydrochlorid e | 1:3.5:10:1 | | Water 30 mL |
| | | | | 15 mg | | | |
| 7 | Polysorbate 80 | Sodium alginate | HPMC | Lipoic acid | 16:4:5:1 | Propy lene glycol | Water 30 mL |
| | | | | 15 mg | | | |
| 8 | Lauryl glucoside | CMC-Na | HPC | Lipoic acid | 14:4:8:1 | Glyce rol | Water 30 mL |
| | | | | 15 mg | | | |
| 9 | Polysorbate 80 | CMC-Na | Poloxamer | Doxycycline | 16:4:8:1 | | Water 30 mL |
| | | | | 15 mg | | | |
| 10 | Polysorbate 80 | CMC-Na | HPMC | Doxycycline | 16:4:8:1 | | Water 30 mL |
| | | | | 15 mg | | | |
| 11 | TPGS (vitamin E-PEG1000 succinate) | CMC-Na | HPMC | Doxycycline | 10:4:6:1 | Propy lene glycol | Water 30 mL |
| | | | | 15 mg | | | |
| 12 | Polysorbate 80 | CMC-Na | Poloxamer | Lipoic acid | 24:6:14: (1:1) | | Water 30 mL |
| | | | | 15 mg | | | |
| | | | | Metformin hydrochlorid e 15 mg | | | |
| 13 | Polysorbate 80 | CMC-Na | Poloxamer | Lipoic acid | 30:6:12: (1:1) | | Water 30 mL |
| | | | | 15 mg | | | |
| | | | | Doxycycline | | | |
| | | | | 15mg | | | |
| 14 | Polysorbate 80 | CMC-Na | Xanthan gum | Resveratrol | 16:4:5:1 | Propy lene glycol | Water 25 mL |
| | | | | 10 mg | | | |
| 15 | Polysorbate 80 | CMC-Na | HPC | Resveratrol | 25:5:6:1 | Propy lene glycol | Water 25 mL |
| | | | | 10 mg | | | |
| 16 | Polysorbate 80 | Sodium starch glycolate | HPMC | Resveratrol | 25:4:6:1 | Glyce rol | Water 25 mL |
| | | | | 10 mg | | | |
| 17 | Polysorbate 80 | PEG2K-(C OOH)₂ | HPMC | Resveratrol | 25:5:6:1 | Propy lene glycol | Water 25 mL |
| | | | | 10 mg | | | |
| 18 | Span 60 | CMC-Na | HPMC | Lipoic acid | 22:5:6:1 | Propy lene glycol | Water 30 mL |
| | | | | 15 mg | | | |
| 19 | Sucrose stearates | CMC-Na | Xanthan gum | Lipoic acid | 2:5:6:1 | Propy lene glycol | Water 30 mL |
| | | | | 15 mg | | | |
| 20 | Azone | CMC-Na | Xanthan gum | Lipoic acid | 2:5:6:1 | Propy lene glycol | Water 30 mL |
| | | | | 15 mg | | | |
| 21 | Polysorbate 80 | CMC-Na | HPMC | Tetracycline hydrochlorid e | 25:5:10: 1 | | Water 40 mL |
| | | | | 40 mg | | | |
| Com parat ive exa mple 1 | Polysorbate 80 | PVP K12 | PVP K30 | Lipoic acid | 4.8:4.8: 20:1 | PEG3 00 | Water 30 mL |
| | | | | 15 mg | | | |
| Com parat ive exa mple 2 | Polysorbate 80 | PVP K12 | HPMC | Metformin hydrochlorid e | 25:6:6:1 | PEG3 00 | Water 30 mL |
| | | | | 15 mg | | | |
| Com parat ive | Polysorbate 80 | PEG4K | HPMC | Resveratrol | 100:25: 25:1 | PEG3 00 | Water 30 mL |
| | | | | 3 mg | | | |
| exa mple 3 | | | | | | | |

In the following, the beneficial effects of the pharmaceutical preparations of the present invention for eye drops were demonstrated with reference to the Experimental examples.

### Experimental example 1: Observation results of the carriers according to the present invention by transmission electron microscope

Transmission Electron Microscope (JEM-2100 Plus, JEOL, Japan)

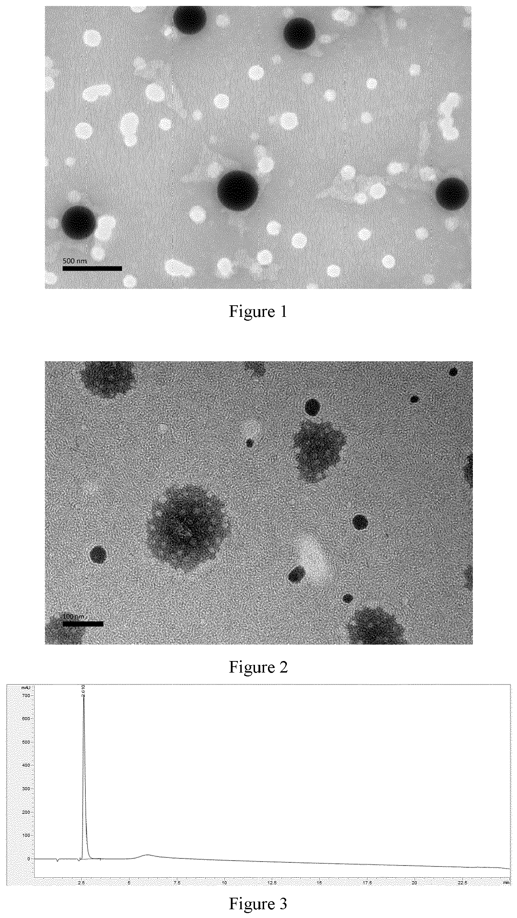

One drop of the solution sample prepared in Example 2 was placed into the copper mesh, and after resting for 5 min, any excess solution sample was removed, then the copper mesh was allowed to dry naturally and placed in the specimen chamber for testing; sample staining: one drop of solution sample was transferred into the copper mesh, and after removing the excess sample from the sample mesh, one drop of 2% phosphomolybdic acid was added, followed by resting for 5 min. Then, the excess solution was removed, and the mesh was naturally dried, and placed in the electron microscope for testing. The results are shown in Figure 1. The sample prepared in Example 9 was observed using the same method, and the results are shown in Figure 2. Spherical structures of 1-100 nm (nanobodies) and spherical structures of 100-2000 nm (nanospheres) composed of nanobodies could be found.

### Experimental example 2: Detection of the particle size, the content, and the stability

### 1. Experimental methods

1 mL of sample prepared in the Example and the Comparative example was moved to the sample cell, and the detection temperature was set to 40 °C. The sample cell was placed into NS-90 nanoparticle size analyser for testing. Each sample was tested three times, and the average of the three test results was represented by the particle size (in terms of light intensity distribution and % percentage) and the polydispersity index (PdI). After testing, the sample was stored in dark, the appearance changes were observed, and then the particle size was retested.

The content of the ophthalmic preparation sample prepared in the present invention was detected by Agilent 1100 high-performance liquid chromatography.

### 2. Experimental results

See the following Table:

**Table 2.**

| Example | Initial particle size (percentage)/PdI/ HPLC content | Storing in a dark place at a certain temperature for 1 month | |
|---|---|---|---|
| | | Particle size (percentage)/PdI | Appearance and content changes |
| 1 | 11.8 nm (71.6%), PdI: 0.519 | Room temperature, 12.50 nm (96.2%), PdI: 0.318; HPLC content: 96.3% | No obvious change was observed. |
| | HPLC content: 97.4% | | |
| 2 | 21.6 nm (94.4%), PdI: 0.206 | Room temperature, 20.2 nm (91.2%), PdI: 0.215; HPLC content: 97.3% | No obvious change was observed. |
| | HPLC content: 99.1% | | |
| 3 | 16.6 nm (98%), PdI: 0.227 | Room temperature, 17.2 nm (92.0%), PdI: 0.215 | No obvious change was observed. |
| | HPLC content: 98.6% | | |
| 4 | 17.1 nm (55.5%) and 513 (36.3%), PdI: 0.795 | Room temperature, 18.5 nm (82.5%), PdI: 0.429 | No obvious change was observed. |
| | HPLC content: 97.8% | HPLC content: 95.3% | |
| 5 | 17.8 nm (98.6%), PdI: 0.222 | 3-8 °C, 19.1 nm (94.2%), PdI: 0.317 | No obvious change was observed. |
| | HPLC content: 97.4% | | |
| 6 | 348 nm (85%), PdI: 0.422 | Room temperature, 178 nm (94.2%), PdI: 0.317 | No obvious change was observed after 1 month; but a small amount of precipitation was observed after 2 months. |
| | HPLC content: 98.4% | | |
| 7 | 25.8 nm (87.4%), PdI: 0.317 | 3-8 °C, 23.6 nm (94.2%), PdI: 0.342; | No obvious change was observed. |
| | HPLC content: 98.1% | | |
| 8 | 31.5 nm (82.9%), PdI: 0.347 | 3-8 °C, 29.5 nm (89.4%), PdI: 0.420 | No obvious change was observed. |
| | HPLC content: 95.2% | | |
| 9 | 16.7 nm (98.1%), PdI: 0.225 | Room temperature, 17.3 nm (95.8%), PdI: 0.261 | No obvious change was observed. |
| | HPLC content: 97.3% | | |
| 10 | 17.2 nm (97.9%), PdI: 0.208; HPLC content: 98.2% | Room temperature, 18.4 nm (93.6%), PdI: 0.265 | No obvious change was observed. |
| 11 | 29.7 nm (89.3%), PdI: 0.382; HPLC content: 95.2% | 3-8 °C, 31.4 nm (53.8%), 246 nm (36.7%), PdI: 0.541 | Cottony substances were observed. |
| 12 | 18.9 nm (45.3%), 302 nm (43.9%), PdI: 0.529 | 3-8 °C, 20.3 nm (44.6%), 243 nm (45.2%), PdI: 0.372 | No obvious change was observed. |
| | HPLC content: 0.486 mg/mL (Metformin), 0.481 mg/mL (Lipoic acid) | | |
| 13 | 20.2 nm (40.7%) and 251.6 nm (61.0%), PdI: 0.701 | 3-8 °C, 21.8 nm (42.1%) and 237 nm (57.0%), PdI: 0.472 | No obvious change was observed. |
| | HPLC content: 0.487 mg/mL (Doxycycline), 0.478 mg/mL (Lipoic acid) | | |
| 14 | 27.5 nm (77.9%), PdI: 0.328 | 3-8 °C, 25.9 nm (89.4%), PdI: 0.245 | No obvious change was observed. |
| | HPLC content: 95.7% | | |
| 15 | 24.5 nm (85.5%), PdI: 0.253 | 3-8 °C, 22.6 nm (91.0%), PdI: 0.302 | No obvious change was observed. |
| | HPLC content: 96.1% | | |
| 16 | 26.2 nm (75.2%), PdI: 0.325 | 3-8 °C, 28.3 nm (86.1%), PdI: 0.264 | No obvious change was observed. |
| | HPLC content: 94.7% | | |
| 17 | 22.7 nm (83.4%), PdI: 0.372 | 3-8 °C, 30.2 nm (79.3%), PdI: 0.427 | No obvious change was observed. |
| | HPLC content: 95.3% | | |
| 18 | 23.7 nm (84.2%), PdI: 0.323 | Room temperature, 25.1 nm (82.7%), PdI: 0.294 | No obvious change was observed. |
| | HPLC content: 96.1% | | |
| 19 | 24.1 nm (81.5%), PdI: 0.357, HPLC content: 95.6% | Room temperature, 26.4 nm (84.2%), PdI: 0.263 | No obvious change was observed. |
| 20 | 27.5 nm (79.6%), PdI: 0.364 | Room temperature, 30.5 nm (81.6%), PdI: 0.407 | No obvious change was observed. |
| | HPLC content: 97.2% | | |
| 21 | 13.4 nm (89.5%), PdI: 0.201; HPLC content: 98.5%; | Room temperature, 15.1 nm (85.7%), PdI: 0.232 | No obvious change was observed. |
| Comp arative examp le 1 | 281.4 nm (85.0%), PdI: 0.224 | Room temperature | Precipitation formed after 2 weeks. |
| Comp arative examp le 2 | 13.9 nm (92.1%), PdI: 0.438 | Room temperature | Precipitation formed after 2 weeks. |
| Comp arative examp le 3 | 14.1 nm (94%), PdI: 0.197 | Room temperature | Precipitation formed after 3 days. |

According to the above results, the pharmaceutical preparation prepared in the present invention had a small particle size, a high content of active pharmaceutical ingredients detected by HPLC, as well as the stable morphology and content after long-term storage; it was indicated that the preparation of the present invention had high encapsulation efficiency and good stability. However, the preparation prepared in the Comparative example using different adjuvants and starting materials from the present invention had poor stability and might experience precipitation or change in a short period of time.

### Experimental example 3. Verifying the effect of the ophthalmic preparation according to the present invention on dry macular degeneration after eye drops

### 1. Experimental methods

Nine SD rats weighing 140-160 g were divided into a control group (3 rats), a model group (3 rats), and a treatment group (3 rats). The model group and the treatment group were injected with 1% sodium iodate (20 mg/kg) through the tail vein of the rats to construct the rat model of dry macular degeneration. The model included the pathological characteristics of dry macular degeneration, and was one of the effective tools to evaluate the new treatment of dry macular degeneration.

24 h after the injection, in the treatment group, 20 µL of the preparation of Example 9 according to the present invention was dropped into the conjunctival sac of the lower eyelid of both eyes, three times a day, for six consecutive days; while in the model group and the control group, rats were treated in the same way as the treatment group except for the normal saline eye drops. 12 h after the last eye drop, the rats were euthanized, and the eyeballs were removed, fixed in 4% Paraformaldehyde, followed by subjecting to conventional paraffin sections and HE staining.

### 2. Experimental results

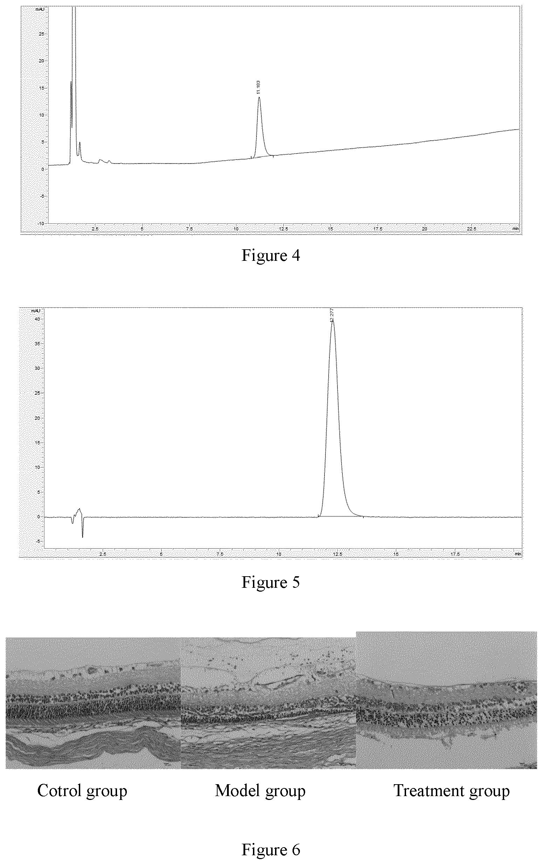

Under an optical microscope, the retina of rats in the control group presented clear layers, neatly arranged cells, and no abnormalities in the cell structure of the outer and inner nuclear layers. The rats in the model group had retinal atrophy, reduced cell numbers in both outer and inner nuclear layers, and disordered cell arrangement. Compared with the model group, the treatment group had an improvement in the degree of retinopathy, an increase in the cell numbers of both outer and inner nuclear layers, together with a relatively neat arrangement of cells (see Figure 6).

The above experimental results demonstrated that the eye drops of the present invention had preventive/therapeutic effects on dry macular degeneration.

In summary, the present invention provided a preparation by eye drop administration, which, by eye drop administration, could carry (encapsulate) an adenylate-activated protein kinase activator and/or an anti-inflammatory active ingredient through the anterior segment and deliver them to the posterior segment of the eye, so as to produce therapeutic effects. The present invention had achieved the goal of preventing and treating dry macular degeneration and retinal light damage by eye drops, solved the technical problems that urgently needed to be solved but still had not been solved in this field, and thereby had extremely excellent clinical values and very positive social significance.

## Claims

1. An ophthalmic preparation for eye drop administration, **characterized in that** it is a preparation composed of an active ingredient for treating an ocular disease as well as an ophthalmic preparation carrier or adjuvant;
the active ingredient for treating the ocular disease is an adenylate-activated protein kinase activator and/or an anti-inflammatory active ingredient;
the ophthalmic preparation carrier or adjuvant contains the following components: a surfactant, an ionic macromolecule, and a solvent.

2. The preparation according to claim 1, **characterized in that** in the ophthalmic preparation carrier or adjuvant, the mass ratio of the surfactants to the ionic macromolecule is: (1-100):(0.1-50); the ratio of the surfactant to the solvent is: every 100 mL of the solvent contains 5-3000 mg of the surfactant.

3. The preparation according to claim 2, **characterized in that** in the ophthalmic preparation carrier or adjuvant, the mass ratio of the surfactants to the ionic macromolecule is (1-30):(3.5-6); the ratio of the surfactant to the solvent is: every 100 mL of the solvent contains 50-2500 mg of the surfactant.

4. The preparation according to any one of claims 1 to 3, **characterized in that** the surfactant is a non-ionic surfactant; preferably, the non-ionic surfactant is Spans, Polysorbates, Poloxamer, alkylglucosides, vitamin E polyethylene glycol succinate (TPGS), sucrose stearates or azone; more preferably, the non-ionic surfactant is Spans, polysorbates, Poloxamer, alkylglucosides, and sucrose stearates.

5. The preparation according to any one of claims 1 to 3, **characterized in that** the ionic macromolecule is selected from at least one of carboxymethyl cellulose (CMC) and its salts, sodium starch glycolate, hyaluronic acid and its salts, Xanthan gum, alginic acid and its salts, and polyethylene glycol diacetate PEG-(COOH)₂.

6. The preparation according to claim 1, **characterized in that** in the ophthalmic preparation carrier or adjuvant, the solvent is a polar solvent, and preferably water.

7. The preparation according to claim 1, **characterized in that** the ophthalmic preparation carrier or adjuvant also contains the following components: adhesive agents and/or cosolvents;
preferably, the adhesive agent is selected from at least one of polyethylene glycol, carbomer, Poloxamer, povidone, hydroxypropyl cellulose, methyl cellulose, hydroxyethyl cellulose, polyvinyl alcohol, Xanthan gum, polyoxyethylene fatty alcohols, hyaluronic acid and its salts or hydroxypropyl methyl cellulose (HPMC); the cosolvent is propylene glycol, glycerol, liquid polyethylene glycol or castor oil; the mass ratio of the adhesive agent to the surfactant is 1 :(0.1-100), and the mass ratio of the cosolvent to the surfactant is (1-10):1;
more preferably, the mass ratio of the adhesive agent to the surfactant is 1:(0.1-6.25), and the mass ratio of the cosolvent to the surfactant is (4.2-10):1.

8. The preparation according to claim 1, **characterized in that** the mass ratio of the surfactant to the active pharmaceutical ingredient for treating the ocular disease is (1-30):(1-2).

9. The preparation according to claim 1, **characterized in that** the adenylate-activated protein kinase activator is at least one of lipoic acid, stereoisomers of lipoic acid, lipoate, stereoisomer salts of lipoic acid, metformin, metformin salts, resveratrol, resveratrol salts; the anti-inflammatory medicament is at least one of doxycycline, doxycycline salts, tetracycline and tetracycline salts.

10. The preparation according to claim 9, **characterized in that** the metformin salt is metformin hydrochloride, and the doxycycline salt is doxycycline hydrochloride, and the tetracycline salt is tetracycline hydrochloride.

11. The preparation according to any one of claims 1 to 10, **characterized in that** the ophthalmic preparation carrier or adjuvent contains nanobodies, which are formed by self-assembly of the components of the ophthalmic preparation carrier or adjuvent; the nanobodies are assembled with active pharmaceutical ingredients for treating the ocular disease.

12. The preparation according to claim 11, **characterized in that** the nanobody is spherical, with a particle size of 1-100 nm; preferably, the particle size of the nanobody is 5-30 nm.

13. The preparation according to claim 12, **characterized in that** it contains nanospheres, which are spherical in shape and have a particle size of 10-2000 nm; the nanospheres are formed by self-assembly of nanobodies; preferably, the particle size of the nanospheres is 100-2000 nm.

14. A method for preparing the preparation according to any one of claims 1 to 13, **characterized in that** it comprises the following steps:
(1) The surfactant and/or the adhesive agent is added to the solvent to prepare a solution;
(2) The active pharmaceutical ingredient for treating the ocular disease and/or the cosolvent is dispersed in the solution obtained in step (1), to which is then added the ionic macromolecular or its solution, and the resultant solution is dispersed and mixed to obtain the initial suspension;
(3) The initial suspension obtained in step (2) is stirred and dispersed or homogenized to obtain the preparation;
preferably, in step (2), the active pharmaceutical ingredient is dissolved in an organic solvent, and then dispersed in the solution obtained in step (1).

15. The method according to claim 14, **characterized in that** the dispersion described in step (2) is selected from at least one of mechanical stirring dispersion, magnetic stirring dispersion, vortex shaking dispersion, shear dispersion, homogeneous dispersion, grinding dispersion, and ultrasonic dispersion.

16. The preparation according to any one of claims 1 to 13 for use in the preparation of medicaments for preventing and treating fundus diseases.

17. The use according to claim 16, **characterized in that** the medicament for preventing and treating fundus diseases is a medicament for preventing and treating dry macular degeneration;
or, the medicament for preventing and treating the damage of light to eye photoreceptor cells or retina.

18. A method for preventing and treating fundus diseases, **characterized in that** the preparation according to any one of claims 1 to 13 is administrated to the patients or individuals at risk at an effective therapeutic amount.

19. The method according to claim 18, **characterized in that** the fundus diseases are dry macular degeneration or the damage of light to eye photoreceptor cells or retina.

20. The method according to claim 18, **characterized in that** the pathway used is eye drop administration.
